# EUROPÄISCHE PATENTSCHRIFT

(11) **EP 1 889 040 B1**
(45) Veröffentlichungstag und Bekanntmachung des Hinweises auf die Patenterteilung: **10.09.2008**
(21) Anmeldenummer: 06742386.3
(22) Anmeldetag: 23.05.2006
(51) Int. Cl.: G01N 21/65, A61B 5/00

(54) **MESSEINRICHTUNG ZUR RAMANSPEKTROMETRISCHEN, NICHTINVASIVEN GLUCOSEBESTIMMUNG**
MEASURING DEVICE FOR A NON-INVASIVE GLUCOSE DETERMINATION USING RAMAN SPECTROMETRY
DISPOSITIF DE MESURE POUR LA DETERMINATION DU GLUCOSE NON INVASIVE PAR SPECTROMETRIE RAMAN

(30) Priorität: 26.05.2005 DE 102005024511
(43) Veröffentlichungstag der Anmeldung: 20.02.2008
(73) Patentinhaber: Hermsdorfer Institut für Technische Keramik e.V., 07629 Hermsdorf (DE)
(72) Erfinder: ARNOLD, Michael, 07743 Jena (DE)
(74) Vertreter: Oehmke, Volker
(86) Internationale Anmeldenummer: PCT/DE2006/000908
(87) Internationale Veröffentlichungsnummer: WO 2006/125430

(56) Entgegenhaltungen:
- US-A- 5 553 616
- US-A- 5 842 995
- US-A- 6 167 290

## Beschreibung

Die Erfindung bezieht sich auf eine Messeinrichtung zur ramanspektrometrischen, nichtinvasiven Glucosebestimmung, enthaltend eine Laserstrahlungsquelle zur Bereitstellung einer, auf einen Gewebeabschnitt gerichteten modulierten Anregungsstrahlung, eine Empfangseinrichtung mit einem wellenlängendispersiven Element zum Empfang einer von dem Gewebeabschnitt abgegebenen Ramanstrahlung, welche die Information über den Glucosegehalt enthält und eine mit der Empfangseinrichtung verbundene Auswerteeinrichtung.

Bekanntermaßen eignen sich ramanspektrometrische Messungen zur nichtinvasiven Glucosebestimmung, da die durch inelastischen Streueffekt hervorgerufenen, positiv und negativ gleichermaßen frequenzverschobenen Raman-Signale der Glucose im Blut noch eine hinreichende Intensität aufweisen, um eine eindeutige Konzentrationsabhängigkeit der Intensität nach dem Lambert-Beer'schen Gesetz feststellen zu können.

Vorteil einer nichtinvasiven Glucosebestimmung ist, dass auf eine unangenehme und für Bluter problematische Blutentnahme verzichtet werden kann. Das Verfahren ist sowohl ambulant als auch in der Klinik durchführbar.

Eine zur Ramanstrahlungsmessung geeignete bekannte faseroptische Sonde gemäß der DE 44 33 305 A1 verwendet am sondenseitigen Ende parallel und zentralsymmetrisch angeordnete Fasern und einen konzentrischen Meniskus als Ein- und Austrittsfenster.
Zwar gelangen am Meniskus reflektierte Strahlungsanteile der Anregungsstrahlung nicht in die zur Detektion der gestreuten Strahlung vorgesehene Empfangsfaser, doch trifft das nicht auf anderswo ungestreut rückreflektierte Strahlungsanteile der Anregungsstrahlung zu.

Aus der US 5 553 616 ist es bekannt, Laserdiodenstrahlung auf einen, in einem Positionierzylinder fixierten Finger zu fokussieren und die gestreute Strahlung über ein aufwendiges Spiegelsystem und eine optische Linse durch ein Notchfilter hindurch auf ein wellenlängendispersives Element zu richten, dem ein Empfänger nachgeordnet ist.
Von Nachteil ist es, dass Notchfilter ausreichend kollimierte Strahlung erfordern, um mit einer guten Performance arbeiten zu können. Da Notchfilter zudem eine hohe Temperaturabhängigkeit aufweisen, sind Einbrüche des Raighleigh-Lichtes nicht ausgeschlossen.
Nachteilig ist außerdem die fehlende Einstellung auf eine spezifische Raman-Linie. Da nicht alle Raman-Linien in jedem Fall auftreten, können sich Fehler ergeben.

Aufgabe der Erfindung ist es, die für ramanspektrometrische Messungen zur nichtinvasiven Glucosebestimmung erforderliche Anregungsstrahlung mit Hilfe einfacher technischer Maßnahmen von der Messstrahlung zu trennen.

Erfindungsgemäß wird diese Aufgabe bei der Messeinrichtung zur ramanspektrometrischen, nichtinvasiven Glucosebestimmung der eingangs genannten Art dadurch erreicht, dass die Anregungsstrahlung über einen Kreisring verteilt, auf einen zylindermantelförmigen Reflektor mit nach innen weisender reflektierender Fläche gerichtet ist, die koaxial eine optische Achse umschließt, auf der ein Fokus für die über den Kreisring verteilte Anregungsstrahlung liegt, und dass eine zur Herstellung eines direkten Kontaktes mit der Oberfläche des Gewebeabschnittes vorgesehene Strahleintrittsfläche der Empfangseinrichtung zu dem Fokus einen vorgegebenen Abstand auf der optischen Achse aufweist, wodurch der Fokus beim Kontakt der Strahleintrittsfläche mit der Oberfläche des Gewebeabschnittes im Gewebeinneren und die Strahleintrittsfläche in einem anregungsstrahlungsfreien Raum liegt.

Der von der Laserstrahlungsquelle bereitgestellte Anregungslaserstrahl kann entweder als zusammenhängender strahlender Abschnitt, oder in Einzelstrahlen zerlegt, als zueinander beabstandete strahlende Abschnitte auf einen Kreisring verteilt sein und wird anschließend mit einstellbarer Eindringtiefe auf einen Anregungsort innerhalb des Gewebeabschnittes, vorzugsweise der Fingerbeere gerichtet, dessen Kapillarblut repräsentative Blutzuckerkonzentrationen aufweist.

Für die Verteilung der Anregungsstrahlung auf den Kreisring sind unterschiedliche technische Lösungen möglich.
In einer ersten Variante eignet sich hierfür ein optischer Mehrfach-Strahlenteiler, der zueinander beabstandete strahlende Abschnitte erzeugt.
Ein über den Kreisring verteilter zusammenhängender strahlender Abschnitt kann z. B. mit einer Faserkapillare bereitgestellt werden, in welche die Anregungsstrahlung eingeleitet wird, wobei die Anregungsstrahlung zunächst koaxial zur optischen Achse geführt und dann zur optischen Achse hin umgelenkt wird.

Eine bevorzugte Ausgestaltung der Erfindung sieht zur Selektion des Raman-Streulichts ein Blaze-Gitter vor, dem ein erster Empfänger nachgeordnet ist.

Wird die Strahleintrittsfläche durch die Stirnseite einer optischen Faser gebildet, lässt sich das Blaze-Gitter auf einfache Weise mit der Strahlaustrittsfläche der optischen Faser in optischen Kontakt bringen.

Ein zweiter optischer Empfänger, auf den die Strahlung einer Eichstrahlungsquelle gerichtet ist, ist als Eichempfänger vorgesehen.

Beide Empfänger sind ausgangsseitig mit einem Differenzverstärker verbunden, an den eine Anzeigeeinrichtung mit bevorzugt integriertem A/D-Wandler zur Darstellung eines digitalisierten Verknüpfungssignals angeschlossen ist.

Vorteilhaft kommt Anregungsstrahlung der Wellenlänge im Bereich um 660 nm zur Anwendung, da Hämoglobin und andere rote Blutfarbstoff-Komponenten vom Porphyrin-Typ bei 660 nm kaum absorbieren und somit bei dieser Wellenlänge sehr streuaktiv sind. Andererseits wird niederfrequentes sichtbares Licht sehr wenig gestreut, wodurch andere, neben dem Raman-Effekt auftretende Farbstreuphänomene weitgehend unterdrückt werden können. Auch andere temporäre Hämoglobinvarianten (Oxy(red)-, Oxy(ox)-, Carboxy(red)-, Carboxy( ox)- oder Cyano- ) ordnen sich hier ein.

Der Aufnahmeort für die zu messende Strahlung befindet sich in einem Raum, der weitgehend frei ist von Anregungsstrahlung, so dass diese nicht in den Empfangskanal gelangen kann. Da somit nur Streustrahlung im Form von Rayleigh-Strahlung und RamanStrahlung empfangen wird, sind im Empfangskanal keine zusätzlichen Maßnahmen zur Abtrennung der Anregungsstrahlung notwendig, wodurch die Streustrahlung auf einfache Weise direkt mit einer optischen Faser abgenommen werden kann. Aufgrund der erfindungsgemäßen Maßnahmen kommt nicht das zufällig einfallende Streulicht zur Auswertung, sondern ausschließlich nichtparalleles Streulicht.
Durch den immer konstanten Abstand zwischen dem Fokus und der Strahleintrittsfläche ist der Gewebeabschnitt prinzipiell in eine Spektrometereinheit integriert, wodurch thermische Interferenzen mit einer anderen Wellenlänge eliminiert werden.

Die Erfindung soll nachstehend anhand der schematischen Zeichnung näher erläutert werden. Es zeigen:
- Fig. 1: einen ersten optischen Aufbau, mit der Anregungsstrahlung auf einen Gewebeabschnitt gerichtet und Messstrahlung aufgenommen wird
- Fig. 2: einen zweiten optischen Aufbau, mit der Anregungsstrahlung auf einen Gewebeabschnitt gerichtet und Messstrahlung aufgenommen wird
- Fig. 3: eine strahlumlenkende Faserkapillare
- Fig. 4: einen Monochromator zur Selektion von Raman-Strahlung aus der Messstrahlung mit angeschlossener Auswerteeinrichtung

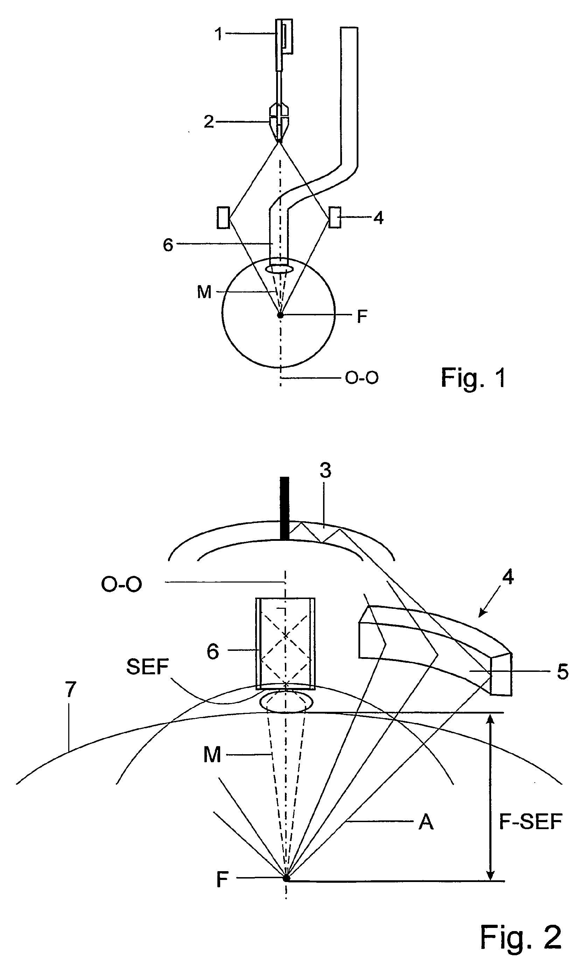

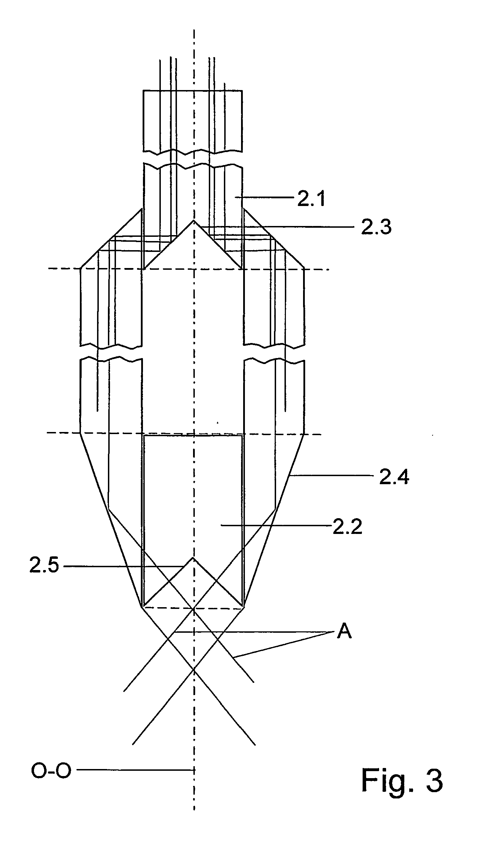

Der in Fig. 1 und 2 dargestellte Aufbau der erfindungsgemäßen Messeinrichtung umfasst als Strahlungsquelle zur Bereitstellung einer Anregungsstrahlung A bevorzugt einen diodengepumpten Festkörperlaser 1, der mit einer Wellenlänge von 671 nm und einem Strahldurchmesser von 1 mm - 1,5 mm Pulse mit einer Pulslänge von 100 ns emittiert. Geeignet sind auch andere gepulste Laseremitter im Bereich um 660 nm, vorausgesetzt, eine zu geringe Kohärenzlänge wirkt sich nicht negativ aus. Mittels einer Faserkapillare 2 (Fig. 1 und 3) oder eines optischen Mehrstrahlenteilers 3 (Fig. 2) wird die Anregungsstrahlung A auf einen Kreisring kontinuierlich oder in diskreten Abschnitten verteilt und auf einen zylindermantelförmigen Reflektor 4 mit nach innen weisender reflektierender Fläche 5 gerichtet.

Die Faserkapillare 2 umschließt gemäß Fig. 3 zwei hintereinander angeordnete optische Fasern 2.1 und 2.2, von denen eine erste einen kegelförmigen Strahlauskopplungsbereich 2.3 aufweist, über den die Anregungsstrahlung A in die Faserkapillare 2 gelangt, in der eine Strahlführung koaxial zur optischen Achse O-O erfolgt. Eine strahlumlenkende Endfläche 2.4 richtet die Anregungsstrahlung A zur optischen Achse 0-0, so dass sie auf einen Kreisring verteilt wird. Eine geeignete kegelförmige Ausnehmung 2.5 im Endbereich der zweiten optischen Faser 2.2 sorgt dabei für eine Beibehaltung der Strahlrichtung nach der Umlenkung zur optischen Achse 0-0.

Die reflektierende Fläche 5 umschließt koaxial eine optische Achse 0-0, auf der ein Fokus F für die über den Kreisring verteilte Anregungsstrahlung A liegt. Entlang der optischen Achse O-O, die mit einer Flächennormalen auf einer als Strahleintrittsfläche SEF dienenden stirnseitigen Fläche einer Empfangsfaser 6 zusammenfällt, besitzt der Fokus F einen vorgegebenen festen Abstand F-SEF von z. B. 3 mm - 5 mm zu der Strahleintrittsfläche SEF.

Wird die Strahleintrittsfläche SEF bei der Messung direkt auf einen Gewebeabschnitt 7, bevorzugt die Fingerbeere aufgesetzt, liegt der Fokus F im Gewebeinneren.

Das für die Glucosebestimmung interessante Raman-Streulicht wird mittels eines sich an die Empfangsfaser 6 anschließenden Blaze-Gitters 8 vom Rayleigh-Streulicht getrennt. Bei einer Anregungswellenlänge von z. B. 660 nm liegen die Stokes-Linien bei Wellenzahlen von 16273 cm⁻¹ und 18458 cm⁻¹, die Antistokes-Linien bei 12256 cm⁻¹ und 14031 cm⁻¹.

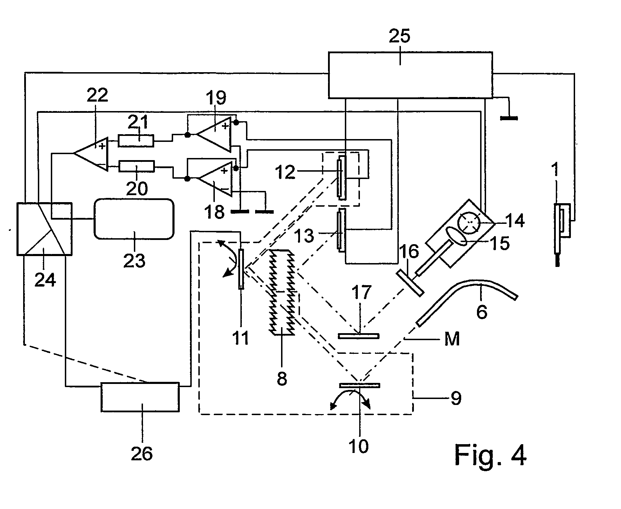

Der in der Messeinrichtung vorgesehene und in Fig. 4 dargestellte Monochromator 9 (gestrichelt umrandet), bestehend aus dem Blaze-Gitter 8, zwei einstellbaren Umlenkspiegeln 10, 11 und einem ersten Empfänger 12 arbeitet ohne Notch-Filter, da die aus der Empfangsfaser 6 austretende Messstrahlung M keinen Anteil der außeraxial unter schrägem Winkel einfallenden Anregungsstrahlung A enthält, sondern neben der Ramanstrahlung nur Rayleigh-Streustrahlung.

Außer dem ersten Empfänger 12 ist ein zweiter Empfänger 13 zur wellenlängen- und intensitätsspezifischen Eichung vorgesehen, auf den die Strahlung einer Eichstrahlungsquelle 14 über einen Kollimator 15, einen Filter 16 und einen Umlenkspiegel 17 gerichtet ist.

Die von den beiden Empfängern 12, 13 gelieferten Ausgangssignale werden über zwei Spannungsfolger 18, 19 mit einer jeweiligen Verstärkung V = + 1 und Eingangswiderstände 20, 21 auf einen Differenzverstärker 22 gebracht, der mit einer Anzeigeeinrichtung 23, wie z. B. einem Balken-Display mit vorzugsweise integriertem A/D-Wandler zur Digitalisierung des Verknüpfungssignals, verbunden ist.

Eine CPU 24 legt zwei logische Grundaufgaben für eine Stromversorgungseinrichtung 25 fest, an welche die beiden Empfänger 12, 13, der Festkörperlaser 1 und die Eichstrahlungsquelle 14 angeschlossen sind.
Zum einen wird die Strahlungsleistung der Eichstrahlungsquelle 14 an die der Messstrahlung M anhand der von den Empfängern 12, 13 gelieferten Signale über eine Dreikanalschaltung angepasst. Andererseits wird der Festkörperlaser 1 über eine Erregung eines nicht dargestellten Laser-Treibers in Abhängigkeit von klinisch festgelegten Messzeiten gesteuert.

Ferner kann die CPU 24 z. B. über eine USB-Schnittstelle mit einem Rechner 26 verbunden werden, um eine Kalibrierung über die beiden einstellbaren Umlenkspiegel 10, 11 vorzunehmen.

Bildet das Blaze-Gitter 8 die erste aktive Zone des als Si-Photodetektor ausgebildeten ersten Empfängers 12, kann die Hautoberfläche praktisch auch in direkten Kontakt mit dem ersten Empfänger 12 gebracht werden.

## Patentansprüche

1. Messeinrichtung zur ramanspektrometrischen, nichtinvasiven Glucosebestimmung, enthaltend eine Laserstrahlungsquelle (1) zur Bereitstellung einer auf einen Gewebeabschnitt (7) gerichteten modulierten Anregungsstrahlung, eine Empfangseinrichtung (12) mit einem wellenlängendispersiven Element (8) zum Empfang einer von dem Gewebeabschnitt abgegebenen Ramanstrahlung, welche die Information über den Glucosegehalt enthält und eine mit der Empfangseinrichtung verbundenen Auswerteeinrichtung, **dadurch gekennzeichnet, dass** die Anregungsstrahlung (A) über einen Kreisring verteilt auf einen zylindermantelförmigen Reflektor (4) mit nach innen weisender reflektierender Fläche (5) gerichtet ist, die koaxial eine optische Achse (O-O) umschließt, auf der ein Fokus (F) für die über den Kreisring verteilte Anregungsstrahlung (A) liegt, und dass eine zur Herstellung eines direkten Kontaktes mit der Oberfläche des Gewebeabschnittes (7) vorgesehene Strahleintrittsfläche (SEF) der Empfangseinrichtung zu dem Fokus (F) einen vorgegebenen Abstand auf der optischen. Achse (O-O) aufweist, wodurch der Fokus (F) beim Kontakt der Strahleintrittsfläche (SEF) mit der Oberfläche des Gewebeabschnittes (7) im Gewebeinneren liegt und die Strahleintrittsfläche (SEF) in einem anregungsstrahlungsfreien Raum liegt.

2. Messeinrichtung nach Anspruch 1, **dadurch gekennzeichnet, dass** die über einen Kreisring verteilte Anregungsstrahlung (A) aus zueinander beabstandeten strahlenden Abschnitten der in Einzelstrahlen zerlegten Anregungsstrahlung (A) besteht.

3. Messeinrichtung nach Anspruch 2, **dadurch gekennzeichnet, dass** zur Erzeugung der über den Kreisring verteilten, zueinander beabstandeten strahlenden Abschnitte ein optischer Mehrfach-Strahlenteiler (3) vorgesehen ist.

4. Messeinrichtung nach Anspruch 1, **dadurch gekennzeichnet, dass** die Anregungsstrahlung zusammenhängend über einen Kreisring verteilt ist.

5. Messeinrichtung nach Anspruch 4, **dadurch gekennzeichnet, dass** zur Erzeugung des über den Kreisring verteilten zusammenhängenden strahlenden Abschnittes eine Faserkapillare (2) vorgesehen ist, die zwei hintereinander angeordnete optische Fasern (2.1, 2.2) umschließt, von denen eine erste einen kegelförmigen Strahlauskopplungsbereich (2.3) aufweist, über den die Anregungsstrahlung (A) in die Faserkapillare (2) gelangt, in der eine Strahlführung koaxial zur optischen Achse 0-0 erfolgt und die eine strahlumlenkende Endfläche (2.4) aufweist, welche die Anregungsstrahlung (A) zur optischen Achse O-O hin umlenkt, so dass sie auf einen Kreisring verteilt wird.

6. Messeinrichtung nach einem der Ansprüche 1 bis 5, **dadurch gekennzeichnet, dass** die Empfangseinrichtung einen ersten Empfänger (12) umfasst, der einem zur Selektion des Raman-Streulichts dienenden Blaze-Gitter (8) nachgeordnet ist.

7. Messeinrichtung nach Anspruch 6, **dadurch gekennzeichnet dass** die Strahleintrittsfläche (SEF) durch die Stirnseite einer optischen Faser gebildet ist, die mit dem Blaze-Gitter (8) im optischen Kontakt steht.

8. Messeinrichtung nach einem der Ansprüche 1 bis 7, **dadurch gekennzeichnet, dass** ein zweiter optischer Empfänger (13) als Eichempfänger vorgesehen ist, auf den die Strahlung einer Eichstrahlungsquelle (14) gerichtet ist.

9. Messeinrichtung nach Anspruch 8, **dadurch gekennzeichnet, dass** die beiden Empfänger (12, 13) ausgangsseitig über die Spannungsfolger (18, 19) mit einem Differenzverstärker (22) verbunden sind, an den eine Anzeigeeinrichtung (23) mit integriertem A/D-Wandler zur Darstellung eines digitalisierten Verknüpfungssignals angeschlossen ist.

10. Messeinrichtung nach einem der Ansprüche 1 bis 9, **dadurch gekennzeichnet, dass** als Laserstrahlungsquelle ein diodengepumpter Festkörperlaser (1) vorgesehen ist, der Anregungsstrahlung (A) mit einer Wellenlänge von 671 nm bereitstellt.

11. Messeinrichtung nach einem der Ansprüche 1 bis 9, **dadurch gekennzeichnet, dass** als Laserstrahlungsquelle ein gepulster Laseremitter vorgesehen ist, der Anregungsstrahlung (A) mit einer Wellenlänge von 660 nm bereitstellt.

## Claims

1. A measuring device for non-invasive glucose determination using Raman spectrometry including a source (1) of laser radiation for providing a modulated excitation beam directed onto a tissue section (7), a receiving device (12) with a wavelength-dispersive element (8) for receiving Raman radiation emitted from the tissue section, which contains information relating to the glucose content, and an analysis device connected to the receiving device, **characterised in that** the excitation beam (A) is distributed over a circular ring and directed onto a cylindrical shell-shaped reflector (4) with an inwardly directed reflective surface (5), which coaxially surrounds an optical axis (O-O) on which a focus (F) for the excitation beam (A) distributed over the circular ring is situated and that a beam entry surface (SEF), which is provided to produce direct contact with the surface of the tissue section (7), of the receiving device has a predetermined spacing from the focus (F) on the optical axis (O-O), whereby, when the beam entry surface (SEF) contacts the surface of the tissue section (7), the focus (F) is situated within the tissue and the beam entry surface (SEF) is situated in a space free of the excitation beam.

2. A measuring device as claimed in claim 1, **characterised in that** the excitation beam (A) distributed over a circular ring consists of radiant sections spaced from one another of the radiation beam split up into individual beams.

3. A measuring device as claimed in claim 2, **characterised in that** an optical multiple beam splitter (3) is provided to produce the radiant sections, which are spaced from one another and are distributed over the circular ring.

4. A measuring device as claimed in claim 1, **characterised in that** the excitation beam is distributed continuously over a circular ring.

5. A measuring device as claimed in claim 4, **characterised in that** a fibre capillary (2) is provided for the production of the continuous radiating section distributed over the circular ring, which capillary surrounds two optical fibres (2.1, 2.2) arranged behind one another, a first one of which has a conical beam catcher region (2.3) via which the excitation radiation (A) passes into the fibre capillary (2), in which the beam is guided coaxially with the optical axis (O-O) and which has a beam-deflecting end surface (2.4), which deflects the excitation beam (A) towards the optical axis (O-O), so that it is distributed onto a circular ring.

6. A measuring device as claimed in one of claims 1 to 5, **characterised in that** the receiving device includes a first receiver (12), which is arranged behind a blaze grating (8) which serves to select the scattered Raman light.

7. A measuring device as claimed in claim 6, **characterised in that** the beam entry surface (SEF) is constituted by the end face of an optical fibre, which is in optical contact with the blaze grating (8).

8. A measuring device as claimed in one of claims 1 to 7, **characterised in that** a second optical receiver (13) constituting a calibration receiver is provided, onto which the beam from a calibration beam source (14) is directed.

9. A measuring device as claimed in claim 8, **characterised in that** the two receivers (12, 13) are connected on the output side via the voltage followers (18, 19) to a differential amplifier (22), connected to which is an indicating device (23) with an integrated A/D converter for producing a digitised linking signal.

10. A measuring device as claimed in one of claims 1 to 9, **characterised in that** a diode-pumped solid body laser (1) is provided as the laser beam source, which provides an excitation beam (A) with a wavelength of 671nm.

11. A measuring device as claimed in one of claims 1 to 9, **characterised in that** a pulsed laser emitter is provided as the laser beam source, which provides a laser beam (A) with a wavelength of 660nm.

## Revendications

1. Dispositif de mesure destiné à la détermination non invasive du glucose par spectrométrie Raman, comportant une source de rayons laser (1) destinée à mettre à disposition un rayonnement d'excitation modulé, dirigé sur une partie de tissu (7), un dispositif de réception (12) avec un élément (8) de dispersion de longueur d'onde, destiné à recevoir un rayonnement Raman émis par la partie de tissu et contenant l'information sur la teneur en glucose, et un dispositif d'analyse relié au dispositif de réception, **caractérisé en ce que** le rayon d'excitation (A), partagé au moyen d'une bague annulaire, est dirigé sur un réflecteur (4) en forme de chemise cylindrique, avec une surface (5) réfléchissante orientée vers l'intérieur, laquelle entoure coaxialement un axe optique (O-O), sur lequel se situe un foyer (F) pour le rayonnement d'excitation (A) partagé au moyen de la bague annulaire, et **en ce qu'**une surface d'entrée des rayons (SEF) du dispositif de réception, prévue pour établir un contact direct avec la surface de la partie de tissu (7), est située sur l'axe optique (O-O) à une distance prédéfinie du foyer (F), moyennant quoi le foyer (F) se situe à l'intérieur du tissu lorsque la surface d'entrée des rayons (SEF) est en contact avec la surface de la partie de tissu (7), et la surface d'entrée des rayons (SEF) se situe dans un espace sans rayonnement d'excitation.

2. Dispositif de mesure selon la revendication 1, **caractérisé en ce que** le rayonnement d'excitation (A), partagé au moyen d'une bague annulaire, est formé par des parties rayonnantes, écartées les unes des autres, du rayonnement d'excitation (A) décomposé en rayons individuels.

3. Dispositif de mesure selon la revendication 2, **caractérisé en ce que,** pour générer les parties rayonnantes, écartées les unes des autres, partagées au moyen de la bague annulaire, il est prévu un séparateur de faisceaux multiple (3) optique.

4. Dispositif de mesure selon la revendication 1, **caractérisé en ce que** le rayonnement d'excitation est partagé sous forme cohérente au moyen de la bague annulaire.

5. Dispositif de mesure selon la revendication 4, **caractérisé en ce que,** pour générer la partie rayonnante cohérente partagée au moyen de la bague annulaire, il est prévu un vaisseau capillaire à fibres (2) qui comporte deux fibres optiques (2.1, 2.2), disposées l'une derrière l'autre, dont l'une comporte une première zone de découplage du rayonnement (2.3) conique, par l'intermédiaire de laquelle le rayonnement d'excitation (A) parvient dans le vaisseau capillaire à fibres (2), dans lequel se produit un guidage des rayons coaxialement à l'axe optique (O-O) et qui comporte une surface finale (2.4) de déviation des rayons, par laquelle le rayonnement d'excitation (A) est dévié vers l'axe optique (O-O), de telle sorte qu'il est partagé sur une bague annulaire.

6. Dispositif de mesure selon l'une quelconque des revendications 1 à 5, **caractérisé en ce que** le dispositif de réception comporte un premier récepteur (12), qui est monté en aval d'un réseau blazé (8) destiné à sélectionner la lumière Raman diffusée.

7. Dispositif de mesure selon la revendication 6, **caractérisé en ce que** la surface d'entrée des rayons (SEF) est formée par la face frontale d'une fibre optique, qui est en contact optique avec le réseau blazé (8).

8. Dispositif de mesure selon l'une quelconque des revendications 1 à 7, **caractérisé en ce qu'**il est prévu un deuxième récepteur optique (13) formant un récepteur d'étalonnage, sur lequel est dirigé le rayonnement d'une source de rayonnement d'étalonnage (14).

9. Dispositif de mesure selon la revendication 8, **caractérisé en ce que** les deux récepteurs (12, 13) sont reliés du côté sortie via un suiveur de tension (18, 19) à un amplificateur différenciateur (22), auquel est raccordé un dispositif d'affichage (23) avec un convertisseur A/N intégré en vue de représenter un signal logique numérisé.

10. Dispositif de mesure selon l'une quelconque des revendications 1 à 9, **caractérisé en ce qu'**il est prévu un laser solide (1) pompé par diodes pour former une source de rayons laser, qui met à disposition un rayonnement d'excitation (A) avec une longueur d'onde de 671 nm.

11. Dispositif de mesure selon l'une quelconque des revendications 1 à 9, **caractérisé en ce qu'**il est prévu un émetteur laser pulsé pour former une source de rayons laser, qui met à disposition un rayonnement d'excitation (A) avec une longueur d'onde de 660 nm.
